# EUROPEAN PATENT SPECIFICATION

(11) **EP 3 288 554 B1**
(45) Date of publication and mention of the grant of the patent: **24.08.2022**
(21) Application number: 16730891.5
(22) Date of filing: 29.04.2016
(51) Int. Cl.: A61K 31/395, A61K 31/496, A61K 35/745, A61K 35/747, A61P 1/00, A61P 1/12

(54) **USE OF ANTIBIOTICS WITH SPECIFIC THERAPEUTIC ACTIVITIES COMBINED WITH THE SIMULTANEOUS USE OF LACTOBACILLI AND/OR BIFIDOBACTERIA WITH A NON-TRANSFERABLE ANTIBIOTIC RESISTANCE, HAVING THE SAME THERAPEUTIC INDICATION**
VERWENDUNG VON ANTIBIOTIKA MIT SPEZIFISCHEN THERAPEUTISCHEN WIRKUNGEN, KOMBINIERT MIT GLEICHZEITIGER VERWENDUNG VON LACTOBACILLI UND/ODER BIFIDOBAKTERIEN MIT EINER NICHT-ÜBERTRAGBAREN ANTIBIOTIKARESISTENZ MIT DERSELBEN THERAPEUTISCHEN INDIKATION
UTILISATION D'ANTIBIOTIQUES EN PRÉSENCE D'ACTIVITÉS THÉRAPEUTIQUES SPÉCIFIQUES COMBINÉE À L'UTILISATION SIMULTANÉE DE LACTOBACILLES ET/OU DE BIFIDOBACTÉRIES DOTÉES D'UNE RÉSISTANCE AUX ANTIBIOTIQUES NON-TRANSFERABLE, PRÉSENTANT LA MÊME INDICATION THÉRAPEUTIQUE

(30) Priority: 30.04.2015 IT MI20150624
(43) Date of publication of application: 07.03.2018
(73) Proprietor: Probiotical S.p.A., 28100 Novara (IT)
(72) Inventor: MOGNA, Giovanni, 28100 Novara (IT)
(74) Representative: Benedetto, Marco
(86) International application number: PCT/IB2016/052436
(87) International publication number: WO 2016/174623

(56) References cited:
- US-A1- 2002 192 201
- DATABASE MEDLINE [Online] US NATIONAL LIBRARY OF MEDICINE (NLM), BETHESDA, MD, US; August 2006 (2006-08), FANIGLIULO LIBERA ET AL: "Role of gut microflora and probiotic effects in the irritable bowel syndrome.", XP002752303, Database accession no. NLM17172187 & FANIGLIULO LIBERA ET AL: "Role of gut microflora and probiotic effects in the irritable bowel syndrome.", ACTA BIO-MEDICA : ATENEI PARMENSIS AUG 2006, vol. 77, no. 2, August 2006 (2006-08), pages 85-89, XP055236933, ISSN: 0392-4203
- VITALI, BEATRICE ET AL: "Immunoregulatory activity of rifaximin associated with a resistant mutant of Bifidobacterium infantis", INTERNATIONAL JOURNAL OF ANTIMICROBIAL AGENTS, vol. 33, no. 4, 2009, pages 387-389, XP002752304, ISSN: 0924-8579, DOI: 10.1016/J.IJANTIMICAG.2008.10.007
- DATABASE MEDLINE [Online] US NATIONAL LIBRARY OF MEDICINE (NLM), BETHESDA, MD, US; January 1993 (1993-01), GIACCARI S ET AL: "Long-term treatment with rifaximin and lactobacilli in post-diverticulitic stenoses of the colon.", XP002752305, Database accession no. NLM8159832
- DATABASE MEDLINE [Online] US NATIONAL LIBRARY OF MEDICINE (NLM), BETHESDA, MD, US; June 2002 (2002-06), BRIGIDI P ET AL: "Effects of rifaximin administration on the intestinal microbiota in patients with ulcerative colitis.", XP002752306, Database accession no. NLM12120885
- DATABASE MEDLINE [Online] US NATIONAL LIBRARY OF MEDICINE (NLM), BETHESDA, MD, US; January 2001 (2001-01), FUJIWARA S ET AL: "Intestinal transit of an orally administered streptomycin-rifampicin-resistant variant of Bifidobacterium longum SBT2928: its long-term survival and effect on the intestinal microflora and metabolism.", XP002752307, Database accession no. NLM11155121
- DATABASE MEDLINE [Online] US NATIONAL LIBRARY OF MEDICINE (NLM), BETHESDA, MD, US; March 2001 (2001-03), FUJIWARA S ET AL: "Establishment of orally-administered Lactobacillus gasseri SBT2055SR in the gastrointestinal tract of humans and its influence on intestinal microflora and metabolism.", XP002752308, Database accession no. NLM11298228
- S FUJIWARA ET AL: "Establishment of orally-administered Lactobacillus gasseri SBT2055SR in the gastrointestinal tract of humans and its influence on intestinal microflora and metabolism", JOURNAL OF APPLIED MICROBIOLOGY., vol. 90, no. 3, 15 March 2001 (2001-03-15) , pages 343-352, XP055236439, GB ISSN: 1364-5072, DOI: 10.1046/j.1365-2672.2001.01251.x
- MOUBARECK C ET AL: "Antimicrobial susceptibility of bifidobacteria", JOURNAL OF ANTIMICROBIAL CHEMOTHERAPY, OXFORD UNIVERSITY PRESS, GB, vol. 55, no. 1, 1 January 2005 (2005-01-01), pages 38-44, XP002346517, ISSN: 0305-7453, DOI: 10.1093/JAC/DKH495
- ZHOU J S ET AL: "Antibiotic susceptibility profiles of new probiotic Lactobacillus and Bifidobacterium strains", INTERNATIONAL JOURNAL OF FOOD MICROBIOLOGY, vol. 98, no. 2, 1 February 2005 (2005-02-01), pages 211-217, XP027663198, ELSEVIER BV, NL ISSN: 0168-1605 [retrieved on 2005-02-01]
- MARIA ROSARIA D'AIMMO ET AL: "Antibiotic resistance of lactic acid bacteria and Bifidobacterium spp. isolated from dairy and pharmaceutical products", INTERNATIONAL JOURNAL OF FOOD MICROBIOLOGY, vol. 115, no. 1, 2 January 2007 (2007-01-02), pages 35-42, XP055236937, NL ISSN: 0168-1605, DOI: 10.1016/j.ijfoodmicro.2006.10.003

## Description

The present invention relates to the use of antibiotics with specific therapeutic activities combined with the simultaneous use of lactobacilli and/or bifidobacteria with a non-transferable antibiotic resistance and having the same therapeutic indication as said antibiotics. In particular, the present invention relates to a composition comprising or, alternatively, consisting of antibiotics suitable for the use in the treatment of acute and chronic intestinal infections due to gram-positive and gram-negative bacteria; diarrheic syndromes; diarrhea due to an imbalance in the intestinal microflora such as for example, summer diarrhea, traveler's diarrhea and enterocolitis; pre- and postoperative prophylaxis of infectious complications in gastrointestinal surgery; gastrointestinal diseases such as irritable bowel syndrome (IBS), constipation and alterations of intestinal microflora, chronic inflammatory intestinal diseases (CIID) (also known as inflammatory bowel diseases (IBD)) which comprise Crohn's disease and ulcerative rectocolitis; said antibiotics being combined with specific strains of bacteria belonging to the species *Bifidobacterium longum* having the same therapeutic indication as said antibiotics (simultaneous or concurrent administration).

Taking antibiotics, which are useful for fighting and eradicating bacterial infections for example in the event of acute and chronic gastrointestinal infections, diarrheic syndromes, irritable bowel syndrome (IBS), chronic inflammatory intestinal diseases (IBD) and infections by *Helicobacter pylori,* often leads to serious side effects to the body, in particular towards the bacterial flora of intestine and mucosae. Antibiotics are also prescribed as a preoperative prophylaxis before surgical or dental procedures. The intended aim, by using antibiotics, is to prevent the proliferation of pathogenic bacteria, which can cause dangerous, even systemic, infections. There exist targeted antibiotics, those which only suppress some specific families of pathogenic bacteria, as well as those "with a broad spectrum activity", being suitable when the body is subjected to infections of unclear etiology, or as a preventive measure.

One of the known unwanted side effects, related to the use of antibiotics, is due to the fact that besides to kill pathogenic bacteria, which are responsible for bacterial infections, antibiotics also destroy the indigenous "good" bacterial flora which colonizes both intestine and mucosae giving rise to, among others, the following symptoms such as: colitis, irritable bowel syndrome (IBS) and intestinal inflammatory disorders characterized by diarrhea, feces in the form of separate hard lumps with occurrence of mucus, abdominal swelling and cramps; *Candida albicans* infections which can affect both intimate (typically, vaginal candidiasis in women) and buccal mucosae with the development of whitish plaques and symptoms such as burning and redness; stomatitis and occurrence of oral aphthae; nausea and stomachache, as well as a weakened immune system resulting in a body more vulnerable to additional infections.

The irritable bowel syndrome (IBS), which, from a clinical point of view, is characterized by the coexistence of abdominal pain and/or swelling as well as alteration of bowels, is often related to an imbalance among the different species of intestinal bacteria. Bowel alterations can be represented by diarrhea, constipation, or both.

In order to limit the adverse effects on the "good" bacterial flora due to antibiotics, it is recommended to take, along with antibiotics, chemical substances belonging to the group of gastro-protective agents which, however, are not devoid of limits and drawbacks or the postponed, not simultaneous, intake of probiotic bacteria at least 8-12 hours after starting antibiotics, in order to prevent antibiotics from also acting against said probiotic bacteria leading to a useless administration of the latter. However, taking probiotic bacteria at least 8-12 hours after the administration of antibiotics is also unable to avoid that a subsequent intake of antibiotics would cause their destruction or inactivation and, thus, in any case prevents them from colonizing the intestinal tract.

Nevertheless, it would be very useful to be able to administrate at the same time, for a specific and well-defined bacterial infection to be treated, an antibiotic having a suitable therapeutic indication effective against said infection, and strains of probiotic bacteria (simultaneous administration) with activity for the same therapeutic indication as the antibiotics being used, in order to preventing or minimizing the effects from an imbalance of the indigenous "good" bacterial flora which colonizes both intestine and mucosae, resulting from the use of antibiotics, thereby assuring a predominance of indigenous bacterial species relative to pathogens so that to avoid all the adverse effects resulting from the antibiotic intake.

The Applicant, following to a long and intense research and development activity, solved the above cited limits and drawbacks of the know art.

Basically, the Applicant found useful to administering a combination consisting of selected antibiotics with a specific and established therapeutic activity against a particular and well-determined infection or disease/disorder to be treated, in association with selected strains of bacteria having a proved antibiotic resistance non-transferable to other species, and effectively used in the treatment for the same therapeutic indication as the antibiotics themselves.

It is an object of the present invention a composition comprising or, alternatively, consisting of an antibiotic, with specific therapeutic indications, in association with strains of bifidobacteria; said bifidobacteria having a proved antibiotic resistance non-transferable to other species, and being further used for the same therapeutic indications as said antibiotics; said composition being for in a method of treatment of:
(i) acute and chronic intestinal infections due to gram-positive and gram-negative bacteria;
(ii) diarrheic syndromes;
(iii) diarrhea due to an imbalance of the intestinal microflora selected from summer diarrhea, traveler's diarrhea and enterocolitis;
(iv) pre- and postoperative prophylaxis of infectious complications in gastrointestinal surgery;
(v) gastrointestinal diseases such as irritable bowel syndrome (IBS), constipation and alterations of intestinal microflora;
(vi) chronic inflammatory intestinal diseases (CIID) (also known as inflammatory bowel diseases (IBD)) comprising Crohn's disease and ulcerative rectocolitis;
(vii) infections by *Helicobacter pylori,* wherein said antibiotic is selected from the group comprising or, alternatively, consisting of rifamycin, rifaximin, rifampicin, rifabutin and rifapentine, wherein said strain of bifidobacteria is *Bifidobacterium longum* W11, access number LMG P-21586, deposited at the Belgian Coordinated Collection of Microorganisms - BCCM LMG, and wherein said antibiotic is simultaneously administered with the bacterial strain *Bifidobacterium longum* W11 LMG P-21586.

Preferred embodiments of the present invention will be evident from the following detailed description. Within the context of the present invention by "compositions of the present invention" is meant food or nutraceutical compositions, or supplement product compositions or medical device compositions (even for oral use), or pharmaceutical compositions.

It is an object of the present invention selected strains of bacteria belonging to the species *Bifidobacterium longum* W11 LMG P-21586, for use in the treatment of gastrointestinal diseases such as irritable bowel syndrome (IBS), diarrhea, constipation, alterations of intestinal microflora, chronic inflammatory intestinal diseases (IBD) and infections by *Helicobacter pylori,* in association with antibiotics, as claimed in the appended claims.

Within the context of the present invention by "association" is meant that the antibiotic, selected from the group comprising or, alternatively, consisting of rifamycins, and the strain of bacteria belonging to the species *Bifidobacterium longum* can be concurrently administered since they can be thoroughly mixed together within the same pharmaceutical formulation, such as for example in a tablet, or hard gel capsule, or packet, or stick, or oil. However, the present invention also encompasses the concept of "association" in which the antibiotic, selected from the group comprising or, alternatively, consisting of rifamycins, and the strain of bacteria belonging to the species *Bifidobacterium longum* can be individually administered, since they are not physically together in the same pharmaceutical formulation, but within a short period of time from each other. For example, the antibiotic can be administered in the form of a tablet (or different pharmaceutical forms) and, shortly thereafter, the bacterial strain can be presented in the form of water-dispersible granules, or capsule, or tablet, or packet, or stick, or oil.

It is an object of the present invention food or nutraceutical compositions, or supplement product compositions, or medical device compositions (even for oral use), comprising a mixture of bacteria which comprises or, alternatively, consists of said strains of bacteria belonging to the species *Bifidobacterium longum,* for use in the treatment of gastrointestinal diseases such as irritable bowel syndrome (IBS), diarrhea, constipation, alterations of intestinal microflora, chronic inflammatory intestinal diseases (IBD) and inflammations due to *Helicobacter pylori,* as claimed in the appended claims. Said compositions being used in association with antibiotics.

It is an object of the present invention pharmaceutical compositions comprising a mixture of bacteria which comprises or, alternatively, consists of said strains of bacteria belonging to the species *Bifidobacterium longum* and antibiotics selected from rifamycins (in particular, rifaximin), for use in the treatment of gastrointestinal diseases such as irritable bowel syndrome (IBS), diarrhea, constipation, alterations of intestinal microflora, chronic inflammatory intestinal diseases (IBD) and inflammations due to *Helicobacter pylori,* as claimed in the appended claims.

The Applicant investigated and selected a number of strains of bacteria belonging to various bacterial species and in particular belonging to the species *Bifidobacterium longum.* Following to an extended research activity, the Applicant achieved to select, among others, the strain of bacteria belonging to the species *Bifidobacterium longum* W11, deposited at the Belgian Coordinated Collection of Microorganisms - BCCM LMG, with access number LMG P-21586.

The present invention relates to biologically pure cultures of the strain of bacteria *Bifidobacterium longum* W11 LMG P-21586 and the use thereof as probiotic for preparing a pharmaceutical composition further comprising, in association, an antibiotic selected from rifamycins (in particular rifaximin), for use in the curative treatment of infections, or syndromes, or diseases, or disorders as described in the above cited items from (i) to (vii); advantageously for use in the treatment of gastrointestinal diseases such as irritable bowel syndrome (IBS), diarrhea, constipation, alterations of intestinal microflora, chronic inflammatory intestinal diseases (IBD) and inflammations due to *Helicobacter pylori.*

The strain of bacteria *Bifidobacterium longum* W11 LMG P-21586 was selected because, besides to show a capability to colonizing the intestinal bacterial flora and adhering to the intestinal cells - characteristics which all render them optimal probiotic agents able to promote a good health of the gastrointestinal tract and restore the functionality impaired by the use of antibiotics - said strain exhibits a surprising and unexpected antibiotic resistance which was shown to be unexpectedly non-transferable to other species. Furthermore, said strain is advantageously suitable for the use in the treatment of gastrointestinal diseases such as irritable bowel syndrome (IBS), diarrhea, constipation, alterations of intestinal microflora, chronic inflammatory intestinal diseases (IBD) and inflammations due to *Helicobacter pylori.*

The Applicant carried out a complete sequencing of the overall genome of the strain of bacteria *Bifidobacterium longum* W11 LMG P-21586 and further conducted a comparative genomic analysis. The results allowed to identifying a genetic locus related to the resistance to the antibiotic rifamycin and semisynthetic derivatives thereof such as rifaximin, rifampicin, rifabutin and rifapentine. The genetic locus is located on the gene rpoB encoding the RNA polymerase beta required for all the transcriptional processes of the bacterium. The analysis of the plasmid DNA revealed the absence of genes involved in rifamycin resistance. Therefore, a locus transferability, which confers resistance to the antibiotic rifamycin cannot occur. Furthermore, the absence of DNA elements indicating a genetic modification of the bacterial strain was observed.

The antibiotic resistance shown by the above cited strain, object of the present invention, was studied and observed towards rifamycin and semisynthetic derivatives thereof such as rifaximin, rifampicin, rifabutin and rifapentine. The antibiotic resistance of a bacterial strain belonging to the genus Lactobacillus and Bifidobacterium, and its non-transferability to other species, along with the ability of said strain to be used for the same therapeutic indication as the antibiotic, collectively represent the fundamental and required characteristics which the strain should possess in order to be selected and used within the context of the present invention. The Applicant further tested and confirmed the non-transferability of said antibiotic resistance in the plasmid content as well.

Therefore, for the first time, the strain of bacteria of the present invention can be formulated along with natural active substances or synthetic or semisynthetic chemical molecules, which all have antibiotic activity, to give a pharmaceutical composition which can be effectively administered, in a safe and advantageous manner, to patients in order to treating and curing bacterial infections without causing side effects typical of treatments with antibiotics alone.

The chemical active substances or natural molecules with antibiotic activity (briefly, antibiotics) are selected from the group comprising or, alternatively, consisting of the family of rifamycins derived from *Streptomices mediterranei,* or synthetic or semisynthetic chemical molecules. Rifamycins usually have a broad-spectrum antibacterial activity against Gram+, Gram- bacteria and mycobacteria and, furthermore, a mechanism of action based on the inhibition of the RNA-nucleotidyltransferase (DNA-dependent RNA-polymerase) by forming very stable complexes 1:1 with said enzyme. Rifamycins generally have a very low toxicity, are poorly absorbed by oral route and are mainly excreted through the bile. The semisynthetic derivatives of rifamycin are selected from rifaximin, rifampicin, rifabutin and rifapentine.

Bacterial cultures of the strain of bacteria *Bifidobacterium longum* W11 LMG P-21586 were firstly produced, according to techniques and apparatuses known to the skilled in the field, in the laboratory and then as industrial preparations. Basically, pure strains were grown at about 37°C for approximately 16 hours in a culture medium TPY based on casein peptone, yeast extract, glucose and mineral salts. From these primary cultures, subsequent subcultures were prepared, in order to increase the number of cells of the starting pure strains until to obtain stock cultures, which were subsequently used as inoculum for industrially producing a bacterial culture of the strain of bacteria *Bifidobacterium longum* W11 LMG P-21586.

Bacterial cultures thus prepared contain on average from about 1×10⁹ to 1×10¹² live cells of *Bifidobacterium longum* W11 LMG P-21586 per gram, as analyzed according to a method based on a flow cytofluorimetric technique, known to the skilled in the field.

Bacterial cultures are known to be tested in a colonization test in the mouse intestine with positive results since the count of the bacterial load of *Bifidobacterium longum* W11 LMG P-21586 shown a value greater than 1×10⁶ live cells per gram of mouse feces.

The efficacy of the bacterial culture and, consequently, also the efficacy of the composition containing said bacterial culture in favoring the gastrointestinal health and in the treatment of intestinal diseases such as diarrhea, traveler's diarrhea, gastroenteritis, constipation, irritable colon, diverticulitis and intestinal inflammatory diseases can be enhanced by adding to said composition food prebiotic fibers in the form of non-digestible oligosaccharides such as, for example, fructooligosaccharides known as FOS, or inulin, or galactooligosaccharides known as GOS, or xylooligosaccharides known as XOS, or arabinoxylooligosaccharides known as AXOS, which are neither absorbed nor hydrolyzed in the first intestinal tract and enhance the activity as well as stimulate the metabolism of the strain of bacteria *Bifidobacterium longum* W11 LMG P-21586 to the detriment of pathogenic bacteria.

Preferred oligosaccharides are mixtures of fructooligosaccharides consisting of a basic unit of a glucose (G) molecule bound to a strand of fructose (F) molecules with general formula GFn, n being less than or equal to 4, having a polymerization degree, namely, a number of monosaccharide units, within 2 and 20. Fructooligosaccharides, which are effectively used in the compositions of the present invention, have a polymerization degree comprised from 2 to 10.

The compositions of the present invention, besides to contain an antibiotic of the rifamycin family and the strain of bacteria *Bifidobacterium longum* W11 LMG P-21586, can comprise several excipients such as sweeteners, for example mannitol, aspartame or sorbitol, flavoring and coloring agents, vitamins, preferably B-group vitamins such as vitamin B1, B2, B6, B9 and B12 and/or D-group vitamins such as vitamin D2 and D3. B-group vitamins are aimed to further support the subject's health by restoring the vitamin component, which is strongly reduced during gastrointestinal diseases and further impaired by the use of antibiotics.

The compositions of the present invention preferably consist of compositions which can be orally administered in the form of capsules, tablets, granules, compressed lozenges and oil, all containing a bacterial load of live and viable cells, due to the sophisticated production technology being used, which is comprised, at the end of the stability period of 24 months, from 1×10⁶ to 1×10¹¹, preferably from 1×10⁷ to 1×10⁹.

The strain of bacteria *Bifidobacterium longum* W11 LMG P-21586 is produced by the Applicant through a technology which allows the cells of the strain to be encapsulated in a protein matrix, forming protein microspheres which contain in their inside the encapsulated cells, making them more resistant both to the gastroduodenal transit and stress affecting the cells during the end product storage. The protein coating prevents from applying the common microbiological techniques for bacterial counting used to assess the bacterial count of a culture. This is because the protein coating is insoluble in diluents and reagents used in the above cited microbiological techniques for bacterial counting, impeding to prepare 10 serial dilutions of the freeze-dried bacterial culture being required. Furthermore, said protein microspheres can encapsulate a variable number of cells (from few to thousands of cells), but by said count only the number of microspheres is determined, making impossible to obtain the effective number of cells. From the above reasons, the Applicant developed a method, which uses a flow cytofluorimetric technique (Internal method 615).

The Applicant carried out some stability tests, by using the internal method 615, in a sample C of the strain of bacteria *Bifidobacterium longum* W11 LMG P-21586 with cells coated with a protein matrix at + 25°C. The results reported in Table 1 surprisingly show almost no mortality, after 9 months at 25°C.

**Table 1**

| **Sample** | **Results T0 months** | **Results T3 months** | **Results T6 months** | **Results T9 months** |
|---|---|---|---|---|
| **C** | 42×10⁹ CFU/g | 42×10⁹ CFU/g | 41×10⁹ CFU/g | 40×10⁹ CFU/g |

The tests for assessing the bacterial count showed that the strain of bacteria *Bifidobacterium longum* W11 LMG P-21586 is resistant to such an extent that it does not undergo a significant mortality, both during its storage and during the technological processes for producing the compositions of the present invention. In fact these bacterial compositions were subjected to stability tests which demonstrated an optimal resistance of the strain of bacteria *Bifidobacterium longum* W11 LMG P-21586 at room temperature of 25°C so that to allow the marketing thereof up to 24 months from the strain production.

The Applicant conducted further tests concerning the osmotic stress stability (extreme conditions of osmotic stress) in sterile water at +25°C at t0 and t7 (after 7 days) in a first sample C1 of viable cells of the strain of bacteria *Bifidobacterium longum* W11 LMG P-21586 coated with a protein matrix (gastro-protected) and a second sample C2 of viable cells of the strain of bacteria *Bifidobacterium longum* W11 LMG P-21586 (non gastro-protected). The results of the reduction are shown in Table 2.

**Table 2**

| **Sample** | **Test method** | **Results t0** | **Results t7 (days)** | **% Mortality** |
|---|---|---|---|---|
| **C1** | Internal method 612 | 105×10⁹ cell/g | 103×10⁹ cell/g | 2% |
| C2 | Internal method 612 | 12×10⁹ cell/g | 5×10⁹ cell/g | 62% |

The compositions of the present invention can further contain prebiotic fibers such as fructooligosaccharides known as FOS, or inulin, or galactooligosaccharides known as GOS, or xylooligosaccharides known as XOS, or arabinoxylooligosaccharides known as AXOS, preferably from 0.5 g to 5 g of fructooligosaccharides FOS, or galactooligosaccharides GOS, per dose, even more preferably from 1 g to 3 g, and/or vitamins, preferably B-group vitamins such as vitamin B1, B2, B6, B9 and B12 and/or D-group vitamins such as vitamin D2 and D3.

An embodiment of the present invention comprises:
- the strain of bacteria *Bifidobacterium longum* W11 LMG P-21586 coated with a protein matrix which allows them to overcome the barrier of gastric juices and bile acids, thus reaching the intestine with a high activity (live and viable cells for a favorable replication),
- a prebiotic fiber selected from fructooligosaccharides, such as the product commercially known as Actilight^{®}, or inulin, or galactooligosaccharides GOS, or xylooligosaccharides XOS, or arabinoxylooligosaccharides AXOS, which is able to cross intact both the stomach and small intestine in order to reach the colon where it becomes a specific nutrient for the Bifidobacteria growth (bifidogenic activity),
- one or more vitamins of group B and/or group D,
- an antibiotic belonging to the group of rifamycins, in particular rifaximin,
- pharmaceutically acceptable excipients, flavors and stabilizers.

With reference to the antibiotics used in the present invention mention can be made, for example, of rifamycin in the form of 200 mg film-coated tablets or granules for oral suspension, 2 g/100 ml (for example Normix^{®}).

## Claims

1. A composition comprising or, alternatively, consisting of: an antibiotic, with specific therapeutic indications, in association with a strain of bifidobacteria; said strain of bifidobacteria having an antibiotic resistance non-transferable to other species, and being also used for the same therapeutic indications as said antibiotics; said composition being for use in a method of treatment of:
(i) acute and chronic intestinal infections due to gram-positive and gram-negative bacteria;
(ii) diarrheic syndromes;
(iii) diarrhea due to an imbalance of the intestinal microflora selected from summer diarrhea, traveler's diarrhea and enterocolitis;
(iv) pre- and postoperative prophylaxis of infectious complications in gastrointestinal surgery;
(v) gastrointestinal diseases such as irritable bowel syndrome (IBS), constipation, and intestinal microflora alterations;
(vi) chronic inflammatory intestinal diseases (CIID) (also known as inflammatory bowel diseases (IBD)) which comprise Crohn's disease and ulcerative rectocolitis;
(vii) infections by *Helicobacter pylori,* wherein said antibiotic is selected from the group comprising or, alternatively, consisting of rifamycin, rifaximin, rifampicin, rifabutin and rifapentine, wherein said strain of bifidobacteria is *Bifidobacterium longum* W11, access number LMG P-21586, deposited at the Belgian Coordinated Collection of Microorganisms - BCCM LMG, and wherein said antibiotic is simultaneously administered with the bacterial strain *Bifidobacterium longum* W11 LMG P-21586.

2. The composition for use according to claim 1, wherein said composition further comprises a prebiotic fiber selected from fructooligosaccharides FOS, or inulin, or galactooligosaccharides GOS, or xylooligosaccharides XOS, or arabinoxylooligosaccharides AXOS; preferably said fructooligosaccharides FOS consist of a basic unit of a glucose (G) molecule bound to a strand of fructose (F) molecules with general formula GFn, n being comprised from 2 to 20, preferably n is comprised from 2 to 10.

3. The composition for use according to claim 1 or 2, wherein said composition further comprises one or more B-group vitamins selected from vitamins B1, B2, B6, B9 and B12 and/or D-group vitamins such as D2 and D3 vitamins.

4. The composition for use according to any one of claims 1-3, wherein the strain of bacteria *Bifidobacterium longum* W11 LMG P-21586 has a concentration comprised from 1×10⁹ to 1×10¹² of live cells per gram.

5. The composition for use according to any one of claims 1-4, wherein said antibiotic and the bacterial strain *Bifidobacterium longum* W11 LMG P-21586 are concurrently administered as a thorough mixture within the same pharmaceutical formulation.

6. The composition for use according to any one of claims 1-4, wherein said antibiotic and the bacterial strain *Bifidobacterium longum* W11 LMG P-21586 are individually administered to a subject within a short period of time from each other, shortly thereafter.

7. The composition for use according to any one of claims 1-6, wherein said antibiotic is rifaximin.

8. The composition for use according to any one of claims 1-7, wherein said composition comprises:
- the strain of bacteria *Bifidobacterium longum* W11 LMG P-21586 coated with a protein matrix, having a concentration comprised from 1×10⁹ to 1×10¹² of live cells per gram;
- the fructooligosaccharide commercially known as Actilight^{®}, or a galactooligosaccharide GOS,
- one or more vitamins of B group and/or D group;
- rifaximin at a concentration of 200 mg; and
- pharmaceutically acceptable excipients, flavors and stabilizers.

## Patentansprüche

1. Zusammensetzung, umfassend oder alternativ bestehend aus: einem Antibiotikum mit spezifischen therapeutischen Indikationen in Verbindung mit einem Stamm von Bifidobakterien; wobei der Stamm von Bifidobakterien eine Antibiotikaresistenz aufweist, die nicht auf andere Spezies übertragbar ist, und auch für dieselben therapeutischen Indikationen wie die Antibiotika verwendet wird; wobei die Zusammensetzung zur Verwendung in einem Verfahren zur Behandlung von:
(i) akuten und chronischen Darminfektionen durch grampositive und gramnegative Bakterien;
(ii) Diarrhoesyndromen;
(iii) Durchfall aufgrund eines Ungleichgewichts der intestinalen Mikroflora, ausgewählt aus Sommerdurchfall, Reisedurchfall und Enterokolitis;
(iv) prä- und postoperativer Prophylaxe infektiöser Komplikationen bei der gastrointestinalen Chirurgie;
(v) Magen-Darm-Erkrankungen wie Reizdarmsyndrom (IBS), Verstopfung und Veränderungen der Darmmikroflora;
(vi) chronischen entzündlichen Darmerkrankungen (CIID) (auch als entzündliche Darmerkrankungen (IBD) bezeichnet), die Morbus Crohn und ulzerative Rektokolitis umfassen;
(vii) Infektionen durch *Helicobacter pylori,* wobei das Antibiotikum ausgewählt ist aus der Gruppe umfassend oder alternativ bestehend aus Rifamycin, Rifaximin, Rifampicin, Rifabutin und Rifapentin, wobei der Bifidobakterienstamm *Bifidobacterium longum* W11, Zugangsnummer LMG P-21586, hinterlegt bei der Belgian Coordinated Collection of Microorganisms - BCCM LMG, ist, und wobei das Antibiotikum gleichzeitig mit dem Bakterienstamm *Bifidobacterium longum* W11 LMG P-21586 verabreicht wird.

2. Zusammensetzung zur Verwendung nach Anspruch 1, wobei die Zusammensetzung ferner eine präbiotische Faser umfasst, die aus Fructooligosacchariden FOS oder Inulin oder Galactooligosacchariden GOS oder Xylooligosacchariden XOS oder Arabinoxylooligosacchariden AXOS ausgewählt ist; wobei die Fructooligosaccharide FOS vorzugsweise aus einer Grundeinheit eines Glucose-(G)-Moleküls bestehen, das an einen Strang von Fructose-(F)-Molekülen mit der allgemeinen Formel GFn gebunden ist, wobei n 2 bis 20 umfasst, wobei n vorzugsweise 2 bis 10 umfasst.

3. Zusammensetzung zur Verwendung nach Anspruch 1 oder 2, wobei die Zusammensetzung ferner ein oder mehrere B-Gruppen-Vitamine, ausgewählt aus den Vitaminen B1, B2, B6, B9 und B12, und/oder D-Gruppen-Vitamine, wie D2- und D3-Vitamine, umfasst.

4. Zusammensetzung zur Verwendung nach einem der Ansprüche 1-3, wobei der Bakterienstamm *Bifidobacterium longum* W11 LMG P-21586 eine Konzentration von 1×10⁹ bis 1×10¹² lebender Zellen pro Gramm aufweist.

5. Zusammensetzung zur Verwendung nach einem der Ansprüche 1-4, wobei das Antibiotikum und der Bakterienstamm *Bifidobacterium longum* W11 LMG P-21586 gleichzeitig als eine gründliche Mischung innerhalb derselben pharmazeutischen Formulierung verabreicht werden.

6. Zusammensetzung zur Verwendung nach einem der Ansprüche 1-4, wobei das Antibiotikum und der Bakterienstamm *Bifidobacterium longum* W11 LMG P-21586 einem Subjekt innerhalb eines kurzen Zeitraums voneinander, kurz danach, individuell verabreicht werden.

7. Zusammensetzung zur Verwendung nach einem der Ansprüche 1-6, wobei das Antibiotikum Rifaximin ist.

8. Zusammensetzung zur Verwendung nach einem der Ansprüche 1-7, wobei die Zusammensetzung Folgendes umfasst:
- den Bakterienstamm *Bifidobacterium longum* W11 LMG P-21586, beschichtet mit einer Proteinmatrix, aufweisend eine Konzentration von 1×10⁹ bis 1×10¹² lebender Zellen pro Gramm;
- das Fructooligosaccharid, im Handel als Actilight^{®} bekannt, oder ein Galactooligosaccharid GOS,
- ein oder mehrere Vitamine der Gruppe B und/oder Gruppe D;
- Rifaximin in einer Konzentration von 200 mg und
- pharmazeutisch annehmbare Hilfsstoffe, Geschmacksstoffe und Stabilisatoren.

## Revendications

1. Composition comprenant ou, alternativement, constituée : d'un antibiotique, avec des indications thérapeutiques spécifiques, en association avec une souche de bifidobactéries ; ladite souche de bifidobactéries ayant une résistance aux antibiotiques non transférable à d'autres espèces, et étant également utilisée pour les mêmes indications thérapeutiques que lesdits antibiotiques ; ladite composition étant destinée à être utilisée dans une méthode de traitement :
(i) des infections intestinales aiguës et chroniques dues à des bactéries Gram-positives et Gram-négatives ;
(ii) des syndromes diarrhéiques ;
(iii) de la diarrhée due à un déséquilibre de la microflore intestinale choisie parmi la diarrhée d'été, la diarrhée du voyageur et l'entérocolite ;
(iv) la prophylaxie pré- et postopératoire des complications infectieuses en chirurgie gastro-intestinale ;
(v) les maladies gastro-intestinales telles que le syndrome de l'intestin irritable (SII), la constipation et les altérations de la microflore intestinale ;
(vi) les maladies inflammatoires chroniques de l'intestin (MICI) (également appelées maladies inflammatoires de l'intestin (MII)), qui comprennent la maladie de Crohn et la rectocolite ulcéro-hémorragique ;
(vii) les infections par *Helicobacter pylori,* dans laquelle ledit antibiotique est choisi dans le groupe comprenant ou, alternativement, est constitué de la rifamycine, la rifaximine, la rifampicine, la rifabutine et la rifapentine, dans lequel ladite souche de bifidobactéries est *Bifidobacterium longum* W11, numéro d'accès LMG P-21586, déposée à la Collection nationale Belge de microorganismes - CCBM LMG, et dans laquelle ledit antibiotique est administré simultanément avec la souche bactérienne *Bifidobacterium longum* W11 LMG P-21586.

2. Composition à utiliser selon la revendication 1, dans laquelle ladite composition comprend de plus une fibre prébiotique choisie parmi les fructooligosaccharides FOS, ou l'inuline, ou les galactooligosaccharides GOS, ou les xylooligosaccharides XOS, ou les arabinoxylooligosaccharides AXOS ; de préférence, lesdits fructooligosaccharides FOS consistent en une unité de base d'une molécule de glucose (G) liée à un brin de molécules de fructose (F) de formule générale GFn, n étant compris entre 2 et 20, de préférence n est compris entre 2 et 10.

3. Composition à utiliser selon la revendication 1 ou 2, dans laquelle ladite composition comprend de plus une ou plusieurs vitamines du groupe B choisies parmi les vitamines B1, B2, B6, B9 et B12 et/ou des vitamines du groupe D telles que les vitamines D2 et D3.

4. Composition à utiliser selon l'une quelconque des revendications 1-3, dans laquelle la souche bactérienne *Bifidobacterium longum* W11 LMG P-21586 a une concentration comprise entre 1 × 10⁹ et 1 × 10¹² de cellules vivantes par gramme.

5. Composition à utiliser selon l'une quelconque des revendications 1-4, dans laquelle ledit antibiotique et la souche bactérienne *Bifidobacterium longum* W11 LMG P-21586 sont administrés simultanément sous forme de mélange homogène au sein de la même formulation pharmaceutique.

6. Composition à utiliser selon l'une quelconque des revendications 1-4, dans laquelle ledit antibiotique et la souche bactérienne *Bifidobacterium longum* W11 LMG P-21586 sont administrés individuellement à un sujet à peu de temps l'un de l'autre, peu après.

7. Composition à utiliser selon l'une quelconque des revendications 1-6, dans laquelle ledit antibiotique est de la rifaximine.

8. Composition à utiliser selon l'une quelconque des revendications 1-7, dans laquelle ladite composition comprend :
- la souche bactérienne *Bifidobacterium longum* W11 LMG P-21586 enrobée d'une matrice protéique, ayant une concentration comprise entre 1 × 10⁹ et 1 × 10¹² de cellules vivantes par gramme ;
- le fructooligosaccharide commercialement connu sous le nom d'Actilight^{®}, ou un galactooligosaccharide GOS,
- une ou plusieurs vitamines du groupe B et/ou du groupe D ;
- la rifaximine à une concentration de 200 mg ; et
- des excipients, des arômes et des stabilisants pharmaceutiquement acceptables.
